(19) Europäisches Patentamt
European Patent Office
Office européen des brevets

(11) Veröffentlichungsnummer : **0 015 867**
**B1**

(12) # EUROPÄISCHE PATENTSCHRIFT

(45) Veröffentlichungstag der Patentschrift : **19.05.82**

(51) Int. Cl.³ : **C 07 C103/78**, A 61 K 49/04

(21) Anmeldenummer : **80730020.7**

(22) Anmeldetag : **06.03.80**

(54) **Trijodierte Isophthalsäurediamide, deren Herstellung und diese enthaltende Röntgenkontrastmittel.**

(30) Priorität : **08.03.79 DE 2909439**

(43) Veröffentlichungstag der Anmeldung : **17.09.80 (Patentblatt 80/19)**

(45) Bekanntmachung des Hinweises auf die Patenterteilung : **19.05.82 Patentblatt 82/20**

(84) Benannte Vertragsstaaten :
**AT BE CH DE FR GB IT LU NL SE**

(56) Entgegenhaltungen :
**DE - A - 2 031 724**
**FR - A - 2 293 919**
**FR - A - 2 354 316**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Speck, Ulrich, Dr.**
**Benediktinerstrasse 50**
**D-1000 Berlin 28 (DE)**
Erfinder : **Blaszkiewicz, Peter, Dr.**
**Rothenburgstrasse 25**
**D-1000 Berlin 41 (DE)**
Erfinder : **Seidelmann, Dieter, Dr.**
**Stierstrasse 14**
**D-1000 Berlin 41 (DE)**
Erfinder : **Klieger, Erich, Dr.**
**Augsburgerstrasse 25**
**D-1000 Berlin 30 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

EP 0 015 867 B1

# Trijodierte Isophthalsäurediamide, deren Herstellung und diese enthaltende Röntgenkrontrastmittel

Die Erfindung betrifft neue trijodierte Isophthalsäurediamide der allgemeinen Formel

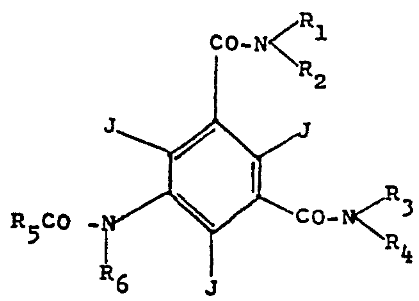

(I),

worin

die Amidreste —CO—N · $R_1R_2$ und —CO—N · $R_2R_4$ voneinander verschieden sind und
$R_1$ ein Wasserstoffatom oder einen niederen Alkylrest,
$R_2$ einen gerad- oder verzweigtkettigen Mono- oder Polyhydroxyalkylrest,
$R_3$ ein Wasserstoffatom oder einen niederen Alkylrest,
$R_4$ einen gerad- oder verzweigtkettigen Mono- oder Polyhydroxyalkylrest,
$R_5$ niedere Alkyl- oder eine niedere Hydroxyalkyl- oder eine Niedere-alkoxy-niedere-alkylgruppe, und
$R_6$ ein Wasserstoffatom oder einen gegebenenfalls hydroxylierten niederen Alkylrest

bedeuten.

Die niederen Alkylreste $R_1$, $R_3$ und $R_6$ enthalten 1-6, vorzugsweise 1-4 Kohlenstoffatome. Beispielsweise genannt seien insbesondere der Methyl-, Äthyl- und Propylrest. Bevorzugt ist der Methylrest.

Der Alkylrest $R_6$ kann auch hydroxyliert sein. Bevorzugt sind 1-3-Hydroxylgruppen im Alkylrest. Beispielsweise genannt seien insbesondere der Hydroxyäthyl- und der Dihydroxypropylrest.

Der Alkylrest im Mono- oder Polyhydroxyalkylrest $R_2$ und $R_4$ kann beliebig lang- und gerad- oder verzweigtkettig sein. Bevorzugt geeignet sind Alkylreste mit 2-8, vorzugsweise mit 2-4 Kohlenstoffatomen. Die Hydroxylgruppen im Alkylrest können als primäre und/oder sekundäre und/oder tertiäre Hydroxylgruppen vorliegen. Der Alkylrest kann 1-5, vorzugsweise 1-3 Hydroxylgruppen enthalten. Als Reste $R_2$ und $R_4$ seien beispielsweise genannt: Trishydroxymethylmethyl, Hydroxyäthyl, insbesondere Dihydroxypropyl.

Der Rest $R_5$ ist ein niederer Alkylrest mit vorzugsweise 1-4 Kohlenstoffatomen. Bevorzugt ist der Methylrest. Ist der Rest $R_5$ hydroxyliert, enthält er vorzugsweise 1-4 C-Atome im Alkylrest und trägt 1-3 Hydroxylgruppen vorzugsweise eine Hydroxylgruppe. Als hydroxylierte Alkylreste $R_5$ seien beispielsweise der Dihydroxypropylrest und vorzugsweise der Hydroxyäthyl- und Hydroxymethylrest genannt. Ist der Alkylrest $R_5$ alkoxyliert, enthält er im Alkylrest 1-3, vorzugsweise ein und im Alkoxyrest 1-3, vorzugsweise 1-2 C-Atome. Genannt sei insbesondere der Methoxymethylrest.

Für die ausreichende Darstellung der harnableitenden Organe, des Gefässsystems, der cerebrospinalen Höhlen und anderer Systeme müssen Röntgenkontrastmittel in höher Dosierung appliziert werden. Das macht die Herstellung hoch konzentrierter Kontrastmittellösungen erforderlich, womit deren physikochemische Eigenschaften wie Löslichkeit, Viskosität und osmotischer Druck stark an Bedeutung gewinnen. So können beispielsweise hochkonzentrierte nichtionische Kontrastmittellösungen aufgrund ihres niederen osmotischen Druckes gegenüber ionischen Kontrastmittellösungen deutliche Vorteile in Bezug auf ihre Verträglichkeit aufweisen.

Röntgenkontrastmittel für die Angiographie, Urographie, Myelographie etc. müssen dazu ausserordentlich gut wasserlöslich sein.

Als erste, gut verträgliche, lösliche und für die praktische Radiologie geeignete nicht ionische schattengebende Substanz ist das Metrizamid (DOS 2 031 724) zu nennen. Beim Metrizamid wird die Löslichkeit ebenso wie beim Ioglunide (DOS 2 456 685) durch einen Polyhydroxyalkylrest bedingt, der über eine Säureamidbindung mit dem trijodierten Aromaten verbunden ist. Substanzen dieses Typs sind neben ihrer schwierigen Herstellbarkeit nicht stabil genug, um in der Hitze sterilisiert werden zu können, und sind auch nicht ausreichend lagerfähig. Für den praktischen Gebrauch in Röntgenkontrastmitteln ist das als schwerwiegender Nachteil anzusehen.

Auch auf Basis der Trijodaminophthalsäureamide haben sich bisher nur einige wenige Derivate ausreichend verträglich und chemisch stabil erwiesen, um als schattengebende Substanzen in Röntgenkontrastmitteln für die intravasale Anwendung geeignet zu sein. In diesen Verbindungen ist die 1- und 3-ständige Amidgruppe symmetrisch substituiert, d.h. beide Carboxylgruppen sind mit dem gleichen Amin amidiert.

Es wurde nun gefunden, dass die aufgezeigten Nachteile ganz vermieden oder mindestens deutlich vermindert werden, wenn der 1- und 3-ständige Amidstickstoff wie in Formel I unterschiedlich substituiert ist. Die unterschiedliche Substitution am Amidstickstoff muss sich nicht allein auf $R_2$ zu $R_4$ beziehen. Im Sinne der vorliegenden Erfindung kann auch von Vorteil sein, dass die Substituenten $R_1$ und $R_3$ von einander verschieden sind.

Die erfindungsgemässen Verbindungen der Formel I sind in Lösung stabil, so dass sie in gebräuchlicher Weise auch durch Erhitzen auf 120 °C bei physiologischem $p_H$-Wert sterilisiert werden können. Die Lösungen haben auch bei hoher Jodkonzentration, verglichen mit den zur Zeit gebräuchlichen ionischen Röntgenkontrastmitteln, einen niedrigen osmotischen Druck, was insbesondere für eine gute lokale Verträglichkeit Voraussetzung ist. Lösungen einiger der neuen Verbindungen sind sehr wenig viskös, was als Bedingung für eine leichte Handhabung gilt. Die Substanzen selbst sind extrem hydrophil, was eine Voraussetzung für ihre gute allgemeine Verträglichkeit ist.

Die erfindungsgemässen Substanzen zeigten im tierexperimentellen Test bei unterschiedlichen Tierspezies sehr gute allgemeine und ausgezeichnete lokale Verträglichkeit, eine sehr gute Herzkreislaufverträglichkeit und nur geringe Neurotoxizität. Darüberhinaus zeigten die erfindungsgemässen Substanzen im in-Vitro-Test eine nur äusserst geringe Interaktion mit Proteinen und nur sehr geringe membranschädigende Wirkung.

Die neuen Verbindungen der Formel I sind aufgrund ihrer guten pharmakologischen Eigenschaften als schattengebende Substanzen auf allen Anwendungsgebieten von wasserlöslichen Röntgenkontrastmitteln, insbesondere für die intravasale, subarachnoidale und unterschiedliche lokale Anwendungen, hervorragend geeignet.

Die Erfindung betrifft somit auch neue Röntgenkontrastmittel auf Basis von Verbindungen der allgemeinen Formel (I). Die Herstellung der neuen Röntgenkontrastmittel auf Basis der erfindungsgemässen Verbindungen der allgemeinen Formel (I) erfolgt in an sich bekannter Weise, z.B. dadurch, dass man die schattengebende Substanz mit den in der Galenik üblichen Zusätzen, z.B. Stabilisatoren wie Natriumedetat, Calcium-di-natriumedetat, physiologisch verträgliche Puffer, Natrium-chlorid u.ä., in eine für die intravenöse Applikation geeignete Form bringt. Die Konzentration der neuen Röntgenkontrastmittel im wässrigen Medium richtet sich ganz nach der röntgendiagnostischen Methode. Die bevorzugten Konzentrationen und Dosierungen der neuen Verbindungen bewegen sich in den Bereichen von 50-400 mg J/ml für die Konzentration und 2-500 ml für die Dosierung. Besonders bevorzugt sind Konzentrationen zwischen 100-400 mg J/ml.

Die Erfindung betrifft ferner ein Verfahren zur Herstellung von Verbindungen der allgemeinen Formel I, dadurch gekennzeichnet, dass man in an sich bekannter Weise

a) eine Verbindung der allgemeinen Formel IIA

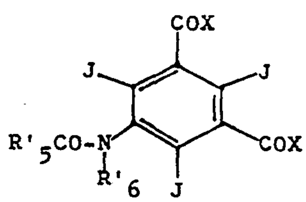

(IIA),

worin

$R'_5 = R_5$, wobei jedoch im Alkylrest anwesende freie Hydroxylgruppen gegebenenfalls funktionell abgewandelt sein können,

$R'_6 = R_6$, wobei jedoch im Alkylrest anwesende freie Hydroxylgruppen gegebenenfalls funktionell abgewandelt sein können,

X = ein reaktiver Säure- oder Esterrest

bedeuten, zunächst mit einer Base der Formel III,

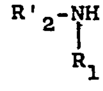

(III),

worin

$R_1$ Wasserstoff oder einen niederen Alkylrest,

$R'_2 = R_2$, wobei im Alkylrest anwesende freie Hydroxylgruppen gegebenenfalls funktionell abgewandelt sein können,

bedeuten und das so erhaltene 5-[$R'_5$—CO—($R'_6$)-amino]-2,4,6-trijod-isophthalsäure-($R'_2$—N—$R_1$)-amidchlorid dann mit einer Base der Formel IV

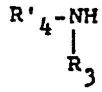

(IV),

worin

$R_3$ Wasserstoff oder einen niederen Alkylrest,

$R'_4 = R_4$, wobei im Alkylrest anwesende freie Hydroxylgruppen gegebenenfalls funktionell abgewandelt können,

bedeuten, umsetzt und gegebenenfalls anschliessend die aromatische Acylaminogruppe mit einem $R'_6$-enthaltenden Alkylierungsmittel N-alkyliert und/oder geschützte Hydroxylgruppen verseift, oder

b) eine Verbindung der allgemeinen Formel IIB

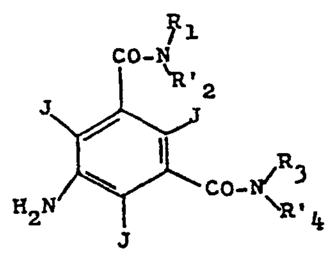

(IIB),

worin

$R_1$ und $R_3$ das gleiche wie oben und $R'_2$ und $R'_4 = R_2$ und $R_4$, wobei jedoch im Alkylrest anwesende freie Hydroxylgruppen funktionell abgewandelt sein können, bedeuten mit einem reaktiven $R'_5$-Carbonsäurederivat N-acyliert, und gegebenenfalls anschliessend mit einem $R'_6$-enthaltenden Alkylierungsmittel N-alkyliert und/oder geschützte Hydroxylgruppen in Freiheit setzt, oder

c) eine Verbindung der allgemeinen Formel IIC

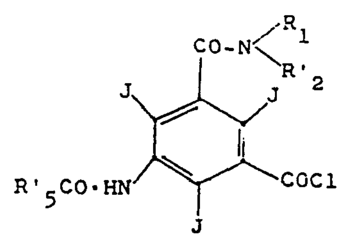

(IIC),

worin

$R_1$, $R'_2$ und $R'_5$ die oben genannte Bedeutung haben, mit einer Base der Formel IV umsetzt und gegebenenfalls anschliessend mit einem $R'_6$-enthaltenden Alkylierungsmittel N-alkyliert und/oder geschützte Hydroxylgruppen in Freiheit setzt.

Als reaktiver Säure- oder Ester-Rest X im Ausgangsprodukt IIA kommt insbesondere ein Halogen, wie —Cl, —Br oder —J infrage. Grundsätzlich durchführbar ist die Umwandlung IIA→I aber auch, wenn X den Azidrest, einen Alkoxycarbonyloxyrest oder den Rest einer reaktiven Estergruppe, z.B. einen üblichen —O—Alkyl, O—Aryl oder $—O—CH_2—C \equiv N$ bedeutet.

Die Umwandlung IIA→I geht bevorzugt von Ausgangsprodukten der Formel IIA mit X in der Bedeutung Cl aus.

Für die Amidierungsreaktion können in den Substituenten $R'_5$ und $R'_6$ anwesende Hydroxylgruppen in freier oder geschützter Form vorliegen. Sollen diese Hydroxylgruppen in geschützter Form vorliegen, kommen alle Hydroxylschutzgruppen infrage, die bekanntermassen für einen intermediären Hydroxylgruppenschutz geeignet sind, d.h. die sich leicht einführen und sich später unter Rückbildung der letztlich gewünschten freien Hydroxylgruppe auch wieder leicht abspalten lassen. Bevorzugt ist der Schutz durch Veresterung z.B. durch Einführung des Benzoyl- oder Acyl-, insbesondere des Acetylrestes. Geeignete Schutzgruppen sind auch Äthergruppen wie z.B. Benzyl-, Di- und Triphenyl-methyl-Äthergruppen sowie Acetal- und Ketalgruppen mit z.B. Acetaldehyd und Aceton.

Die Amidierung der beiden 1- und 3-ständigen Carboxylgruppen erfolgt stufenweise. Die beiden Amidierungsreaktionen erfolgen in einem geeigneten Lösungsmittel bei 0-100 °C, vorzugsweise bei 20-80 °C. Geeignete Lösungsmittel sind u.a. polare Lösungsmittel. Beispielsweise genannt seien Wasser, Dioxan, Tetrahydrofuran, Dimethylformamid, Dimethylacetamid, Hexametapol u.ä. und deren Gemische. Da die Amidierungsreaktion exotherm verläuft, ist es gegebenenfalls zweckmässig, das Reaktionsgemisch leicht zu kühlen, um die Reaktionstemperatur auf etwa 50 °C halten zu können. Da mit der Amidierungsreaktion Chlorwasserstoff frei wird, der zwecks Neutralisation gebunden werden muss, benötigt man pro Säurechloridgruppe zwei Äquivalente Base, zweckmässigerweise im Überschuss von ca. 10 %. Zur praktischen Durchführung wird das gelöste Ausgangsprodukt IIA in erster Stufe umgesetzt mit 2 Äquivalente der Base III oder mit einem Äquivalent der Base III und einem Äquivalent einer von III verschiedenen Base, die dann als Protonenakzeptor dient. Das Monoamid wird zur Vermeidung von Nebenreaktionen bei der Weiterverarbeitung zweckmässigerweise in üblicher Weise isoliert und in zweiter Stufe in analoger Weise mit der Base IV zum Diamid umgesetzt.

Erfolgt die erste Amidierungsstufe mit Base III in Gegenwart eines Protonenakzeptors, kann die zweite Amidierungsstufe mit Base IV gegebenenfalls auch ohne Isolierung des primär entstandenen Monoamids im Eintopf-Verfahren durchgeführt werden.

Als Protonenakzeptoren zur Neutralisation des bei der Amidierung entstehenden Chlorwasserstoffs verwendet man vorteilhaft tertiäre Amine, wie z.B. Triäthylamin, Tributylamin oder Pyridin.

Die im Reaktionsverlauf anfallenden anorganischen oder organischen Salze werden in bekannter Weise abgetrennt, vorteilhaft z.B. mit Hilfe üblicher Ionenaustauscher-Säulen oder durch Filtration über bekannte Adsorbentien wie z.B. Diaion oder Amberlite® XAD-2 und 4.

Die N-Alkylierung der 5-Acylamino-gruppe zur Einführung des Restes $R_6$ erfolgt ebenfalls nach dem Fachmann bekannten Methoden, beispielsweise in dem man das Diamid in alkalischer Lösung mit dem entsprechenden $R'_6$-Alkylhalogenid, vorzugsweise $R'_6$-Alkylbromid, bei Raumtemperatur umsetzt.

Macht es der Reaktionsverlauf erforderlich, in den Substituenten $R_2$ und/oder $R_4$ und/oder $R_5$ und/oder $R_6$ anwesende freie Hydroxylgruppen intermediär zu schützen, so erfolgt dies nach üblichen Methoden durch leicht reversible Gruppen. Die Einführung solcher Schutzgruppen kann z.B. erfolgen durch Veresterung (z.B. Einführung eines vorzugsweisen Acetylrestes oder Benzoylrestes) oder durch Verätherung (z.B. Einführung des Triphenylmethylrestes).

Der Hydroxylgruppenschutz kann auch durch Katalisierung oder Acetalisierung, z.B. mittels Acetaldehyd, Aceton oder Dihydropyran, erreicht werden.

Die spätere Abspaltung der intermediär eingeführten Schutzgruppen unter Freisetzung der letzlich gewünschten Hydroxylgruppen erfolgt ebenfalls nach Methoden, die dem Fachmann allgemein geläufig sind. So kann die Abspaltung der Schutzgruppen ohne besondere Reaktionsstufe mit der Aufarbeitung und Isolierung der Umsetzungsprodukte erfolgen. Sie kann aber auch in üblicher Weise in einer getrennten Reaktionsstufe durchgeführt werden. Acylschutzgruppen können beispielsweise durch alkalische und Acetal-, Ketal- oder Ätherschutzgruppen durch saure Hydrolyse abgespalten werden.

Sollen die erfindungsgemässen Verbindungen der Formel I über die Verfahrensvariante b) hergestellt werden, erfolgt die Acylierung der aromatischen Aminogruppe im entsprechenden Ausgangsprodukt der allgemeinen Formel IIB ebenfalls nach an sich bekannten Verfahren, in dem man z.B. das Amin in einem inerten Lösungsmittel, wie z.B. Pyridin, DMA, DMF u.ä. bei Temperaturen von 0 °C bis Raumtemperatur mit einem reaktiven $R'_5 \cdot CO$-Säurederivat, vorzugsweise mit dem entsprechenden Säurehalogenid, insbesondere Säurechlorid oder aber auch mit einem entsprechenden Säureanhydrid, vorzugsweise in Gegenwart eines sauren Katalysators, wiz z.B. $H_2SO_4$, umsetzt.

In Abwandlung zur Verfahrensvariante a) kann es zwecks Vermeidung von Nebenprodukten vorteilhaft sein, die Einführung der ersten Amidgruppe schon in einer frühen Vorstufe vorzunehmen. Zur Herstellung der erfindungsgemässen Verbindungen der Formel I geht man dann zweckmässigerweise von einem Monoamid der allgemeinen Formel IIC aus (es ist identisch mit dem Primärprodukt gemäss Verfahrensvariante a), das wie oben beschrieben, mit einer Base der Formel IV amidiert wird. Die sich gegebenenfalls anschliessende Alkylierung der aromatischen Acylaminogruppe und die Abspaltung von anwesenden Hydroxylschutzgruppen erfolgt ebenfalls wie oben dargestellt.

Das Ausgangsprodukt der allgemeinen Formel IIA erhält man aus dem bekannten 5-Amino-2,4,6-trijod-isophthalsäuredichlorid in dem man zunächst die Aminogruppe in bekannter Weise mit dem entsprechenden Säurechlorid $R'_5$-COCl (worin $R'_5$ die oben angegebene Bedeutung hat) in einem geeigneten Lösungsmittel, z.B. Dimethylacetamid oder Dimethylformamid bei 0-10 °C acyliert.

Soll $R_6$ ein gegebenenfalls hydroxylierter niederer Hydroxyalkylrest sein, erfolgt die N-Alkylierung, wie bereits oben ausgeführt, ebenfalls nach an sich bekannten Methoden. Ist $R_6$ im Verfahrensprodukt der Formel I eine unsubstituierte Alkylgruppe, z.B. die Methylgruppe, ist es vorteilhaft, dass diese bereits im Ausgangsprodukt der Formel IIA enthalten ist. Ist $R_6$ im Verfahrensprodukt der Formel I jedoch ein Hydroxyalkylrest, erfolgt die Einführung zweckmässigerweise nach den Amidierungsreaktionen. Soll der Rest $R_6$ aber schon im Ausgangsprodukt der Formel IIA vorgegeben sein, ist es vorteilhaft, wenn die in $R_6$ enthaltenen Hydroxylgruppen intermediär geschützt werden.

Das Ausgangsprodukt der Formel IIB erhält man z.B. ebenfalls aus 5-Amino-2,4,6-trijod-isophthalsäuredichlorid, in dem man die beiden Säurechloridgruppen, wie oben für die Umsetzung IIA → I beschrieben, amidiert, oder aus 5-Nitro-isophthalsäure-monomethylester, wie beispielsweise für 5-Amino-2,4,6-trijod-isophthalsäure-[(2,3-diacetoxy-propyl)-(2,3-diacetoxy-N-methyl-propyl)]-diamid in Beispiel 7 beschrieben.

Das Ausgangsprodukt der Formel IIC erhält man zweckmässigerweise auch aus dem leicht zugänglichen 5-Nitro-isophthalsäuremono-methylester. Durch Aminolyse der Methylestergruppe wird zunächst der Amidrest —N · $R_1R'_2$ bzw. —N · $R_3R'_4$ eingeführt. Liegen anwesende Hydroxylgruppen im Amidrest in freier Form vor, werden diese vorzugsweise in üblicher Weise z.B. als O-Acetat, geschützt. Die anschliessende Reduktion der Nitrogruppe zur aromatischen Aminogruppe erfolgt ebenfalls nach an sich bekannten Methoden, z.B. mit Raney-Nickel in Gegenwart eines niederen Alkohols wie Methanol oder Äthanol bei normalem oder erhöhtem Druck. Das so erhaltene 5-Amino-isophthalsäure-monoamid wird nun in üblicher Weise trijodiert und die freie Carboxylgruppe in die Säurehalogenidgruppe, vorzugsweise in die —COCl-Gruppe, überführt. Anschliessend wird die aromatische Aminogruppe in üblicher Weise mit einem reaktiven $R'_5$-Säurederivat, wie oben beschrieben, zum Ausgangsprodukt der allgemeinen Formel IIC N-acyliert.

Beispiel 1

5-(S-2-Hydroxy-propionylamino)-2,4,6-trijod-isophthalsäure-[(2,3-dihydroxy-N-methyl-propyl)-(2-hydroxy-1-hydroxymethyläthyl)]-diamid

20,8 g (27,2 mMol) 5-(S-2-Acetoxy-propionylamino)-2,4,6-trijod-isophthalsäure-(2-hydroxy-1-hydroxymethyläthyl)-amidchlorid werden in 60 ml trockenem DMF gelöst, bei Raumtemperatur 5,8 g (55 mMol) 2,3-Dihydroxy-N-methyl-propylamin hinzugefügt und 3 Stunden auf 40 °C erwärmt. Das Lösungsmittel wird im Vakuum abgedampft, der ölige Rückstand in 80 ml Wasser gelöst, mit 35 ml konzentriertem wässrigem Ammoniak auf pH 11 gestellt und 3 Stunden bei Raumtemperatur gerührt. Die Lösung wird dann auf ca. 50 ml eingedampft und über einen Anionen- und einen Kationenaustauscher entsalzt. Beim Eindampfen des wässrigen Eluats werden 16,3 g = 76 % d.Th. der gewünschten Verbindung erhalten.

Beispiel 2

5-(S-2-Hydroxy-propionylamino)-2,4,6-trijod-isophthalsäure-[(2-hydroxy-äthyl)-(2-hydroxy-1-hydroxymethyläthyl)]-diamid

11,5 g (15 mMol) 5-(S-2-Acetoxy-propionylamino)-2,4,6-trijod-isophthalsäure-(2-hydroxy-1-hydroxymethyläthyl)-amid-chlorid werden in 45 ml trockenem DMF gelöst, 2,94 g (40 mMol) Äthanolamin hinzugefügt und drei Stunden bei Raumtemperatur gerührt. Das Lösungsmittel wird im Vakuum abgedampft, das verbleibende Öl in 20 ml Wasser gelöst, mit 40 ml wässrigem konzentriertem Ammoniak 3 Stunden bei Raumtemperatur gerührt, die Lösung dann im Vakuum auf ca. 30 ml eingedampft und über einen Anionen- und einen Kationenaustauscher entsalzt. Das eingedampfte wässrige Eluat ergibt 7,4 g (66 % d.Th.) der gewünschten Verbindung.

Beispiel 3

5-(S-2-Hydroxy-propionylamino)-2,4,6-trijod-isophthalsäure-[(2-hydroxy-N-methyl-äthyl)-(2-hydroxy-1-hydroxymethyläthyl)]-diamid

11,5 g (15 mMol) 5-(S-2-Acetoxy-propionylamino)-2,4,6-trijod-isophthalsäure-(2-hydroxy-1-hydroxymethyläthyl)-amid-chlorid werden in 45 ml trockenem DMF gelöst, 3 g (40 mMol) N-Methyläthanolamin hinzugefügt und 3 Stunden bei Raumtemperatur gerührt. Das Lösungsmittel wird im Vakuum abgedampft, das verbleibende Öl in 20 ml Wasser gelöst, mit 40 ml konzentriertem wässrigem Ammoniak 3 Stunden bei Raumtemperatur gerührt, die Lösung dann im Vakuum auf ca. 30 ml eingeengt und über einen Anionen- und einen Kationenaustauscher entsalzt. Das eingedampfte wässrige Eluat ergibt 8,6 g (75 % d.Th.) der gewünschten Verbindung

Beispiel 4

5-(S-2-Hydroxy-propionylamino)-2,4,6-trijod-isophthalsäure-[(2,3-dihydroxy-N-methyl-propyl)-(2,3-dihydroxy-propyl)]-diamid

a) 5-(S-2-Acetoxy-propionylamino)-2,4,6-trijod-isophthalsäure-(2,3-dihydroxy-propyl)-amid-chlorid

14,2 g (20 mMol) 5-(S-2-Acetoxy-propionylamino)2,4,6-trijod-isophthalsäure-dichlorid werden in 60 ml trockenem DMF gelöst, bei Raumtemperatur eine Lösung von 3,83 g (42 mMol) 2,3-Dihydroxy-propylamin, gelöst in 15 ml DMF, eingetropft und 80 Minuten nachgerührt. Die Reaktionslösung wird dann auf 25 ml eingeengt, in 250 ml auf 80 °C erwärmtes Dioxan eingerührt, vom Niederschlag des Hydrochlorids dekantiert, auf 50 ml eingeengt und in 400 ml Methylenchlorid eingerührt. Der feine feste Niederschlag wird bei 50 °C im Vakuum getrocknet. Ausbeute 10,2 g (67 % d.Th.).

b) 5-(S-2-Hydroxy-propionylamino)-2,4,6-trijod-isophthalsäure-[(2,3-dihydroxy-N-methyl-propyl)-(2,3-dihydroxypropyl)]-diamid

10,2 g (13,4 mMol) 5-(S-2-Acetoxypropionylamino)-2,4,6-trijod-isophthalsäure-(2,3-dihydroxy-propyl)-amid-chlorid werden in 30 ml trockenem DMF gelöst, eine Lösung von 2,95 g (28,14 mMol) 2,3-Dihydroxy-N-methyl-propylamin in 8 ml DMF bei Raumtemperatur hinzugefügt und 1 Stunde nachgerührt. Die Reaktionslösung wird eingeengt, der ölige Rückstand in 50 ml Wasser gelöst und mit 12 ml konzentriertem wässrigem Ammoniak 4 Stunden bei Raumtemperatur gerührt. Danach wird mit Salzsäure auf pH 7 eingestellt, auf 100 ml verdünnt und an 250 ml Amberlite XAD-4 entsalzt. Ausbeute 7 g (66 % d.Th.).

## Beispiel 5

5-(S-2-Hydroxy-propionylamino)-2,4,6-trijod-isophthalsäure-[(2-hydroxy-äthyl)-(tris-hydroxymethyl-methyl)]-diamid

a) 5-(S-2-Acetoxy-propionylamino)-2,4,6-trijod-isophthalsäure-(tris-hydroxymethyl-methyl)-amid-chlorid

20 g (28,2 mMol) 5-(S-2-Acetoxy-propionylamino)-2,4,6-trijod-isophthalsäure-dichlorid werden zusammen mit 6,82 g (56,4 mMol) Tris(hydroxymethyl)-methylamin in 100 ml Dioxan 72 Stunden am Rückfluss gekocht, heiss filtriert und das Filtrat eingedampft. Ausbeute 9,8 g (43 % d.Th.).

b) 5-(S-2-Hydroxy-propionylamino)-2,4,6-trijod-isophthalsäure-[(2-hydroxy-äthyl)-(tris-hydroxymethyl-methyl)]-diamid

2,9 g (3,76 mMol) 5-(S-2-Acetoxy-propionylamino)-2,4,6-trijod-isophthalsäure-(tris-hydroxymethyl-methyl)-amid-chlorid und 0,46 g (7,52 mMol) Äthanolamin werden zusammen in 10 ml trockenem DMF 3 Stunden bei Raumtemperatur gerührt. Die Reaktionslösung wird dann in 200 ml Methylenchlorid eingerührt, die Fällung abfiltriert, getrocknet, in 50 ml Wasser gelöst und an 100 ml Amberlite XAD-4 entsalzt. Ausbeute 1,9 g (67 % d.Th.).

## Beispiel 6

5-Methoxyacetylamino-2,4,6-trijod-isophthalsäure-[(2,3-dihydroxy-N-methyl-propyl)-(2,3-dihydroxy-propyl)]-diamid

a) 5-Methoxyacetylamino-2,4,6-trijod-isophthalsäure-dichlorid

121,2 g (1,6 Mol) Methoxyessigsäure werden in 400 ml trockenem DMF gelöst, auf 0 °C gekühlt, innerhalb von 60 Minuten 116 ml (1,6 Mol) Thionylchlorid eingetropft, 30 Minuten bei 10 °C nachgerührt, 138,3 g (0,4 Mol) 5-Amino-2,4,6-trijod-isophthalsäuredichlorid eingetragen und 20 Stunden bei Raumtemperatur nachgerührt. Die Reaktionslösung wird in 10 lit. Wasser eingerührt, der Niederschlag abgesaugt, mit Wasser gewaschen, erneut in Wasser ausgerührt, abgesaugt und bei 50 °C im Vakuum getrocknet. Ausbeute 198 g (0,296 Mol) = 74 % d.Th.

b) 5-Methoxyacetylamino-2,4,6-trijod-isophthalsäure-(2,3-dihydroxy-propyl)-amid-chlorid

50 g (75 mMol) 5-Methoxyacetylamino-2,4,6-trijod-isophthalsäure-dichlorid werden in 200 ml trockenem DMF gelöst, 18 g (97,3 mMol) Tributylamin hinzugefügt, auf 60 °C erwärmt und bei dieser Temperatur eine Lösung von 7,5 g (82,4 mMol) 2,3-Dihydroxy-propylamin in 50 ml DMF eingetropft. Es wird 2 Stunden bei 60 °C nachgerührt, dann auf 100 ml eingeengt und in 1,2 lit Methylenchlorid eingerührt. Der Niederschlag, der sich dabei bildet, wird abgesaugt, getrocknet, zweimal mit je 500 ml Essigester aufgekocht und heiss filtriert. Das eingedampfte Filtrat ergibt 40,5 g (56 mMol) = 74,5 % d.Th. des gewünschten Produktes als farblosen Feststoff.

c) 5-Methoxyacetylamino-2,4,6-trijod-isophthalsäure-[(2,3-dihydroxy-N-methyl-propyl)-(2,3-dihydroxypropyl)]-diamid

50 g (69,2 mMol) 5-Methoxyacetylamino-2,4,6-trijod-isophthalsäure-(2,3-dihydroxy-propyl)-amid-chlorid werden in 150 ml trockenem DMF gelöst und bei Raumtemperatur eine Lösung von 7,25 g (69,2 mMol) 2,3-Dihydroxy-N-methyl-propylamin und 12,85 g (69,2 mMol) Tributylamin in 80 ml DMF eingetropft. Nach zweistündigem Rühren bei Raumtemperatur wird auf ca. 100 ml eingeengt und das Konzentrat in 2 lit. Methylenchlorid eingerührt. Der Niederschlag wird abgesaugt, getrocknet, in 250 ml Wasser gelöst, der pH auf 7 eingestellt, mit 5 g Aktivkohle 30 Minuten gerührt, filtriert und das Filtrat an

1,5 lit Amberlite XAD-4 entsalzt. Nach dem Eindampfen des wässrigen Eluats erhält man das Produkt als farblosen Feststoff. Ausbeute 44,4 g (56,05 mMol) = 81 % d.Th.

## Beispiel 7

a) 5-Nitro-isophthalsäure-(2,3-dihydroxy-N-methyl-propyl)-monoamid

22,5 g (100 mMol) 5-Nitro-isophthalsäure-monomethylester werden 20,5 g (200 mMol) 2,3-Dihydroxy-N-methyl-propylamin in 120 ml Methanol 24 Stunden am Ruckfluss gekocht. Die Reaktionslösung wird dann auf ca. 70 ml eingeengt und 700 ml IN Salzsäure eingetropft, wobei sich die Verbindung als Feststoff abscheidet. Es wird abgesaugt, mit Wasser gewaschen und bei 50 °C im Vakuum getrocknet. Ausbeute 25 g (83,8 mMol) = 83,8 % d.Th.

Die Verbindung wird auch erhalten, wenn zu einer Lösung von 30 g (286 mMol) 2,3-Dihydroxy-N-methyl-propylamin und 43,7 g (520 mMol) Natriumhydrogencarbonat in 200 ml Wasser bei 5 °C eine Lösung von 63,3 g (260 mMol) 5-Nitro-isophthalsäure-monomethylesterchlorid, gelöst in 200 ml Aceton, hinzugefügt wird und man das zunächst entstehende Esteramid mit Natronlauge verseift. Ausbeute 62,6 g (210 mMol) = 80,7 % d.Th.

b) 5-Nitro-isophthalsäure-(2,3-dihydroxy-N-methyl-propyl)-amid-methylester

25 g (83,8 mMol) 5-Nitro-isophthalsäure-(2,3-dihydroxy-N-methyl-propyl)-monoamid werden in 100 ml Methanol gelöst, 0,3 ml (5,5 mMol) konz. $H_2SO_4$ hinzugefügt und 5 Stunden bei Raumtemperatur gerührt. Die Reaktionslösung wird dann mit 1 g (12,8 mMol) Natriumacetat abgepuffert, auf ca. 30 ml eingeengt und das Konzentrat in 300 ml Wasser eingerührt. Der Niederschlag wird abgesaugt, mit Wasser gewaschen und getrocknet. Ausbeute 25 g (80,5 mMol) = 96 % d.Th.

Die Verbindung ist auch aus 5-Nitro-isophthalsäure-monomethylesterchlorid darstellbar:

30 g (123,2 mMol) 5-Nitro-isophthalsäure-monomethylesterchlorid werden in 100 ml Dioxan gelöst und eine Lösung von 13 g (123,2 mMol) 2,3-Dihydroxy-N-methyl-propylamin und 22,8 g (123,2 mMol) Tributylamin in 80 ml Dioxan eingetropft. Nach 5-stündigem Rühren bei Raumtemperatur wird die Lösung auf ca. 80 ml eingeengt und in 500 ml Wasser eingerührt. Der Niederschlag wird abgesaugt, mit Wasser gewaschen und getrocknet. Ausbeute: 33,5 g (107,2 mMol) = 87 % d.Th.

c) 5-Nitro-isophthalsäure [(2,3-dihydroxy-propyl)-(2,3-dihydroxy-N-methyl-propyl)]-diamid

30 g (96,1 mMol) 5-Nitro-isophthalsäure-(2,3-dihydroxy-N-methyl-propyl)-amid-methylester werden in 150 ml Methanol zusammen mit 10,5 g (115,3 mMol) 2,3-dihydroxypropylamin 20 Stunden am Rückfluss gekocht. Die Reaktionslösung wird eingedampft und das verbleibende Öl ohne weitere Reinigung in die nächste Stufe eingesetzt. Die Umsetzung ist gemäss Dünnschichtchromatografie quantitativ.

d) 5-Nitro-isophthalsäure-[(2,3-diacetoxy-propyl)-(2,3-diacetoxy-N-methyl-propyl)]-diamid

36 g (96 mMol) 5-Nitro-isophthalsäure-[(2,3-dihydroxy-propyl)-(2,3-dihydroxy-N-methyl-propyl)]-diamid werden in einem Gemisch aus 70 ml Eisessig + 70 ml Acetanhydrid gelöst, 0,5 ml (9,2 mMol) konz. $H_2SO_4$ hinzugefügt und 5 Stunden bei Raumtemperatur gerührt. Die Reaktionslösung wird dann mit 1,5 g (18,3 mMol) Natriumacetat abgepuffert, eingeengt und der Rückstand in 500 ml Wasser eingerührt. Der Niederschlag wird abgesaugt, mit Wasser gewaschen und getrocknet. Ausbeute: 47,6 g (88,3 mMol) = 92 % d.Th.

e) 5-Amino-2,4,6-trijod-isophthalsäure-[(2,3-diacetoxy-propyl)-(2,3-diacetoxy-N-methyl-propyl)]-diamid

20 g (37,1 mMol) 5-Nitro-isophthalsäure-[(2,3-diacetoxypropyl)-(2,3-diacetoxy-N-methyl-propyl)]-diamid werden in 120 ml Methanol gelöst, mit 1,5 g Raney-Nickel versetzt und 3 Stunden unter einem Wasserstoffdruck von 100 at bei Raumtemperatur hydriert. Der Katalysator wird abfiltriert, das Filtrat auf 80 °C erwärmt, 50 ml 2N Salzsäure hinzugefügt und im Verlaufe von 1 Stunde 66,7 ml (133,3 mMol) 2N $NaJCl_2$-Lösung eingetropft. Es wird 3 Stunden bei 80 °C nachgerührt. Bei Abkühlung auf Raumtemperatur scheidet sich das Produkt als Feststoff ab. Ausbeute: 23,5 g (26,5 mMol) = 71,3 % d.Th.

f) 5-Methoxyacetylamino-2,4,6-trijod-isophthalsäure-[(2,3-dihydroxy-propyl)-(2,3-dihydroxy-N-methyl-propyl)]-diamid

10 g (113 mMol) Methoxyessigsäure werden in 50 ml DMA gelöst, auf 0 °C gekühlt und bei 0-5 °C 8,2 ml (113 mMol) Thionylchlorid eingetropft. Die Lösung wird 1 Stunde bei 10 °C gerührt, dann 50 g (56,4 mMol) 5-Amino-2,4,6-trijod-isophthalsäure [(2,3-diacetoxy-propyl)-(2,3-diacetoxy-N-methyl-propyl)]-diamid eingetragen und 20 Stunden bei Raumtemperatur nachgerührt. Die Lösung wird eingedampft und

der Rückstand in 1 lit. Wasser eingerührt. Der Niederschlag wird abgesaugt, mit Wasser gewaschen, in 300 ml Wasser suspendiert und bei 50 °C solange mit konz. wässrigem Ammoniak gerührt, bis eine klare Lösung entstanden ist. Diese wird eingedampft, der Rückstand in 250 ml Wasser gelöst, die Lösung auf pH 7 eingestellt und an 1,5 lit. Adsorber Amberlite XAD-4 entsalzt. Ausbeute : 35 g (44,2 mMol) = 78,4 % d.Th.

Beispiel 8

a) 5-Nitro-isophthalsäure-(2,3-diacetoxy-N-methyl-propyl)-mono-amid

29,8 g (100 mMol) 5-Nitro-isophthalsäure-(2,3-dihydroxy-N-methyl-propyl)-monoamid werden in einem Gemisch aus 50 ml Eisessig + 50 ml Acetanhydrid gelöst, mit 0,3 ml (5,5 mMol) konz. $H_2SO_4$ versetzt und 3 Stunden bei Raumtemperatur gerührt. Die Lösung wird dann mit 1 g (12,8 mMol) Natriumacetat abgepuffert, eingeengt und in 1 lit. Wasser eingerührt. Das Produkt fällt dabei als Feststoff aus. Dieser wird abgesaugt, mit Wasser gewaschen und bei 50 °C im Vakuum getrocknet. Ausbeute : 35,2 g (92 mMol) = 92 % d.Th.

b) 5-Amino-2,4,6-trijod-isophthalsäure-(2,3-diacetoxy-N-methyl-propyl)-monoamid

20 g (52,3 mMol) 5-Nitro-isophthalsäure-(2,3-diacetoxy-N-methyl-propyl)-monoamid werden in 100 ml Methanol in Gegenwart von 1,5 g Raney-Nickel 3 Stunden unter einem Wasserstoffdruck von 100 at bei Raumtemperatur hydriert. Der Katalysator wird dann abfiltriert, das Filtrat auf 80 °C erwärmt, mit 50 ml 1N Salzsäure versetzt und anschliessend im Verlaufe von 1 Stunde 86,5 ml (173 mMol) 2N $NaJCl_2$-Lösung eingetropft. Es wird 2 Stunden bei 80 °C nachgerührt und auf Raumtemperatur gekühlt. Das Produkt scheidet sich als Feststoff ab. Ausbeute : 27,8 g (38 mMol) = 73 % d.Th.

Die Reduktion der Nitrogruppe kann auch unter Normaldruck in Gegenwart von 10 %-igem Pd/-C-Katalysator durchgeführt werden. Zu diesem Zweck werden 20 g (52,3 mMol) 5-Nitroisophthalsäure-(2,3-diacetoxy-N-methyl-propyl)-monoamid in 100 ml Methanol gelöst, 1,5 g 10 %-iger Pd/C-Katalysator hinzugefügt und 5 Stunden bei Raumtemperatur hydriert. Nach der Abtrennung des Katalysators wird die Hydrierlösung in der oben beschriebenen Weise jodiert. Ausbeute : 26 g (35,6 mMol) = 68 % d.Th.

c) 5-Amino-2,4,6-trijod-isophthalsäure-(2,3-diacetoxy-N-methyl-propyl)-amid-chlorid

109,5 g (150 mMol) 5-Amino-2,4,6-trijod-isophthalsäure-2,3-diacetoxy-N-methyl-propyl)-monoamid werden in 600 ml Toluol gelöst, bis zur Beendigung der Abscheidung von Wasser am Wasserabscheider gekocht, dann auf 40 °C gekühlt und portionsweise 33,3 g (160 mMol) $PCl_5$ eingetragen. Die Suspension wird anschliessend 2 Stunden bei 60 °C und 5 Stunden bei Raumtemperatur gerührt, das Toluol dann unter vermindertem Druck abdestilliert und der feste Rückstand durch Ausrühren mit Methylenchlorid und Benzin gereinigt. Ausbeute : 96,5 g (129 mMol) = 86 % d.Th.

d) 5-Methoxyacetylamino-2,4,6-trijod-isophthalsäure-(2,3-diacetoxy-N-methyl-propyl)-amid-chlorid

30 g (0,4 Mol) Methoxyessigsäure werden in 100 ml DMA gelöst, auf 0 °C gekühlt und bei 0-3 °C 29 ml (0,4 Mol) Thionylchlorid eingetropft. Die Lösung wird anschliessend 1 Stunde bei 10 °C gerührt, dann 150 g (0,2 Mol) 5-Amino-2,4,6-trijod-isophthalsäure-(2,3-diacetoxy-N-methyl-propyl)-amid-chlorid eingetragen und 15 Stunden bei Raumtemperatur nachgerührt. Die Reaktionslösung wird unter vermindertem Druck eingeengt, das verbleibende Öl in 3 lit. Methylenchlorid eingerührt, der Niederschlag abgesaugt, mit Benzin ausgerührt und getrocknet. Ausbeute : 133 g (162 mMol) = 81 % d.Th.

e) 5-Methoxyacetylamino-2,4,6-trijod-isophthalsäure-[(2,3-dihydroxy-propyl)-(2,3-dihydroxy-N-methyl-propyl)]-diamid

50 g (61 mMol) 5-Methoxyacetylamino-2,4,6-trijod-isophthalsäure-(2,3-diacetoxy-N-methyl-propyl)-amid-chlorid werden in 150 ml trockenem DMF gelöst und bei Raumtemperatur eine Lösung von 6,4 g (70 mMol) 2,3-Dihydroxy-propylamin und 11,3 g (61 mMol) Tributylamin in 80 ml DMF eingetropft. Nach zweistündigem Rühren bei Raumtemperatur wird auf ca. 100 ml eingeengt und das Konzentrat in 2 lit. Methylenchlorid eingerührt. Der Niederschlag wird abgesaugt, getrocknet, in 300 ml Wasser gelöst, bei 50 °C 2 Stunden mit 50 ml konzentriertem wässrigem Ammoniak gerührt, weitgehend eingedampft, erneut in 300 ml Wasser gelöst, neutral gestellt und an 1,5 lit. Adsorber Amberlite XAD-4 entsalzt. Ausbeute : 38 g (48 mMol) = 78,7 % d.Th.

Beispiel 9

5-Methoxyacetylamino-2,4,6-trijod-isophthalsäure-[(2,3-dihydroxy-N-methyl-propyl)-(2-hydroxy-1-hydroxymethyläthyl)]-diamid

a) 5-Methoxyacetylamino-2,4,6-trijod-isophthalsäure-(2-hydroxy-1-hydroxymethyläthyl)-amid-chlorid

50 g (75 mMol) 5-Methoxyacetylamino-2,4,6-trijod-isophthalsäure-dichlorid werden in 200 ml trockenem DMF gelöst und bei Raumtemperatur eine Lösung von 14,3 g (157 mMol) 1,3-Dihydroxypropyl-amin in 50 ml trockenem DMF eingetropft. Die Temperatur steigt vorübergehend auf 45 °C an. Es wird 1 Stunde bei Raumtemperatur nachgerührt. Die Reaktionslösung wird auf 100 ml eingeengt und in 800 ml auf 80 °C erwärmtes Dioxan eingerührt, wobei das Hydrochlorid des 1,3-Dihydroxypropylamins ölig ausfällt. Die Dioxanlösung wird von diesem dekantiert, auf ein Konzentrat eingeengt und dieses in 1 lit. Methylenchlorid eingerührt. Das Produkt fällt dabei als Feststoff aus, wird abgesaugt und bei 50 °C im Vakuum getrocknet. Ausbeute : 39,8 g (mMol) = 73,4 % d.Th.

b) 5-Methoxyacetylamino-2,4,6-trijod-isophthalsäure-[(2,3-dihydroxy-N-methyl-propyl)-(2-hydroxy-1-hydroxymethyläthyl)]-diamid

30,7 g (42,5 mMol) 5-Methoxyacetylamino-2,4,6-trijod-isophthalsäure-(2-hydroxy-1-hydroxymethyläthyl)-amid-chlorid werden in 200 ml trockenem DMF gelöst und bei Raumtemperatur eine Lösung von 10 g (89 mMol) 2,3-Dihydroxy-N-methyl-propylamin in 40 ml trockenem DMF dazugetropft. Es wird 3 Stunden bei Raumtemperatur nachgerührt, die Reaktionslösung dann auf ca. 80 ml eingeengt und in 1 lit. Methylenchlorid eingetropft. Der flockige Niederschlag wird abgesaugt, in 100 ml Wasser gelöst, Reste organischen Lösungsmittels durch erneutes Eindampfen entfernt und schliesslich die wässrige Lösung an Amberlite XAD-4 entsalzt. Ausbeute 23,3 g (29,5 mMol) = 69 % d.Th.

## Beispiel 10

5-(N-Methoxyacetyl-methylamino)-2,4,6-trijod-isophthalsäure-[(2,3-dihydroxy-N-methyl-propyl)-(2,3-dihydroxy-propyl)]-diamid

a) 5-(N-Methoxyacetyl-methylamino)-2,4,6-trijod-isophthalsäuredichlorid

30 ml (400 mMol) Methoxyessigsäure werden in 100 ml DMA gelöst, auf 0 °C gekühlt, 29 ml (400 mMol) $SOCl_2$ eingetropft, 30 Minuten bei dieser Temperatur nachgerührt, eine Lösung von 61 g (100 mMol) 5-methylamino-2,4,6-trijod-isophthalsäuredichlorid in 200 ml DMA eingetropft und 20 Stunden bei Raumtemperatur nachgerührt. Die Reaktionslösung wird in 4 lit. Wasser eingerührt, der Niederschlag abgesaugt, in 2 lit. Essigester gelöst, nacheinander mit je 250 ml gesättigter Bicarbonatlösung, gesättigter Kochsalzlösung und Wasser ausgeschüttelt, die Essigesterphase über $Na_2SO_4$ getrocknet und eingedampft. Ausbeute : 61,4 g (90 mMol) = 90 % d.Th.

b) 5-(N-Methoxyacetyl-methylamino)-2,4,6-trijod-isophthalsäure(2,3-dihydroxy-propyl)-amid-chlorid

58,3 g (85,5 mMol) 5-(N-Methoxyacetyl-methylamino)-2,4,6-trijod-isophthalsäure-dichlorid werden in 200 ml trockenem DMF gelöst und bei Raumtemperatur eine Lösung von 16,4 g (180 mMol) 2,3-Dihydroxy-propylamin in 50 ml DMF eingetropft. Es wird 1 Stunde nachgerührt, die Reaktionslösung dann auf 100 ml eingeengt, in 800 ml auf 80 °C erwärmtes Dioxan eingerührt, vom Niederschlag des Aminhydrochlorids dekantiert, die Dioxan-lösung eingeengt und in 1 lit. Methylenchlorid eingerührt. Der Niederschlag wird abgesaugt, mit Methylenchlorid nachgewaschen und bei 50 °C im Vakuum getrocknet. Ausbeute : 53,8 g (73 mMol) = 85,4 % d.Th.

c) 5-(N-Methoxyacetyl-methylamino)-2,4,6-trijod-isophthalsäure-[(2,3-dihydroxy-N-methyl-propyl)-(2,3-dihydroxypropyl)]-diamid

53,8 g (73 mMol) 5-(N-Methoxyacetyl-methylamino)-2,4,6-trijod-isophthalsäure(2,3-dihydroxy-propyl)-amid-chlorid, gelöst in 300 ml trockenem DMF, werden bei Raumtemperatur mit einer Lösung von 18 g (171 mMol) 2,3-Dihydroxy-N-methyl-propylamin in 50 ml DMF versetzt. Nach einstündigem Rühren bei Raumtemperatur wird auf 100 ml eingeengt und in 1 lit. Methylenchlorid eingerührt. Dabei entsteht ein öliger Niederschlag, der in Wasser gelöst und an Amberlite XAD-4 entsalzt wird. Ausbeute : 49 g (61 mMol) = 83,6 % d.Th.

## Beispiel 11

5-(N-Methoxyacetyl-methylamino)-2,4,6-trijod-isophthalsäure-[(2,3-dihydroxy-N-methyl-propyl)-(2-hydroxy-1-hydroxymethyläthyl)]-diamid

a) 5-(N-Methoxyacetyl-methylamino)-2,4,6-trijod-isophthalsäure-(2-hydroxy-1-hydroxymethyläthyl)-amid-chlorid

68,2 g (100 mMol) 5-(N-Methoxyacetyl-methylamino)-2,4,6-trijod-isophthalsäure-dichlorid werden zusammen mit 22,8 g (250 mMol) 1,3-Dihydroxy-propylamin (Serinol) in 400 ml Dioxan 10 Stunden auf 60 °C erwärmt. Der Inhalt des Reaktionskolbens wird heiss filtriert und das Filtrat auf 100 ml eingeengt. Aus dieser Lösung kristallisiert das Produkt aus. Ausbeute : 54 g (73,3 mMol) = 73,3 % d.Th.

b) 5-(N-Methoxyacetyl-methylamino)-2,4,6-trijod-isophthalsäure-[(2,3-dihydroxy-N-methyl-propyl)-(2-hydroxy-1-hydroxymethyläthyl)]-diamid

20 g (27,2 mMol) 5-(N-Methoxyacetyl-methylamino)-2,4,6-trijod-isophthalsäure-(2-hydroxy-1-hydroxymethyläthyl)-amid-chlorid werden in 80 ml trockenem DMF gelöst, eine Lösung von 5,72 g (54,4 mMol) 2,3-Dihydroxy-N-methyl-propylamin in 20 ml DMF eingetropft und 1 Stunde bei Raumtemperatur nachgerührt. Die Reaktionslösung wird auf 50 ml eingeengt, in 500 ml Methylenchlorid eingerührt, der Niederschlag abgetrennt, in 150 ml Wasser gelöst und an ca. 600 ml Adsorber Amberlite XAD-4 entsalzt. Ausbeute : 17 g (21 mMol) = 77,5 % d.Th.

## Beispiel 12

5-(N-Acetyl-2-hydroxyäthylamino)-2,4,6-trijod-isophthalsäure-[(2,3-dihydroxy-propyl)-(2-hydroxy-1-hydroxymethyläthyl)]-diamid

a) 5-Acetylamino-2,4,6-trijod-isophthalsäure-(2-hydroxy-1-hydroxymethyl-äthyl)-amid-chlorid

50 g (78,4 mMol) 5-Acetylamino-2,4,6-trijod-isophthalsäure-dichlorid werden in 70 ml DMF gelöst, die Lösung mit 500 ml Dioxan verdünnt, auf 60 °C erwärmt und bei dieser Temperatur 15 g (165 mMol) 1,3-Dihydroxy-propylamin (Serinol), gelöst in 70 ml DMF, eingetropft. Nach 30 Minuten wird die warme Reaktionslösung vom Niederschlag dekantiert, dieser mit 150 ml Dioxan ausgekocht, die Dioxanlösungen vereinigt, eingedampft, das verbleibende Öl in 800 ml Methylenchlorid eingerührt, der dabei fallende Niederschlag abgesaugt und bei 50 °C im Vakuum getrocknet. Ausbeute : 38 g (54 mMol) = 69 % d.Th.

b) 5-Acetylamino-2,4,6-trijod-isophthalsäure-[(2,3-dihydroxypropyl)-(2-hydroxy-1-hydroxymethyläthyl)]-diamid

8,84 g (97 mMol) 2,3-Dihydroxy-propylamin werden in 85 ml DMF gelöst, 30,5 g (44 mMol) 5-Acetylamino-2,4,6-trijod-isophthalsäure-(2-hydroxy-1-hydroxymethyläthyl)-amid-chlorid hinzugefügt und eine Stunde bei Raumtemperatur gerührt. Die Reaktionslösung wird eingedampft, das verbleibende Öl in 800 ml Methylenchlorid eingerührt, der Niederschlag abgetrennt, in Wasser gelöst und an 600 ml Amberlite XAD-4 entsalzt. Ausbeute : 25 g (33,5 mMol) = 76 % d.Th.

c) 5-(N-Acetyl-2-hydroxyäthylamino)-2,4,6-trijod-isophthalsäure-[(2,3-dihydroxy-propyl)-(2-hydroxy-1-hydroxymethyläthyl)]-diamid

21,7 g (29 mMol) 5-Acetylamino-2,4,6-trijod-isophthalsäure-[(2,3-dihydroxy-propyl)-(2-hydroxy-1-hydroxymethyläthyl)]-diamid werden in 17,4 ml (87 mMol) 5N NaOH bei Raumtemperatur gelöst, 7,25 g (58 mMol) Bromäthanol hinzugefügt und 5 Stunden bei Raumtemperatur gerührt. Die Reaktionslösung wird dann mit Salzsäure auf pH 7 gestellt, eingedampft, der Rückstand an Äthanol aufgenommen, das ungelöst bleibende Salz abfiltriert, das Filtrat eingedampft, der Rückstand in Wasser gelöst und an Amberlite XAD-4 entsalzt. Ausbeute : 16 g (20,2 mMol) = 70 % d.Th.

## Beispiel 13

5-(N-Acetyl-2-hydroxyäthylamino)-2,4,6-trijod-isophthalsäure-[(2,3-dihydroxy-N-methyl-propyl)-(2-hydroxy-1-hydroxymethyläthyl)]-diamid

a) 5-Acetylamino-2,4,6-trijod-isophthalsäure-[(2,3-dihydroxy-N-methyl-propyl)-(2-hydroxy-1-hydroxymethyläthyl)]-diamid

28,6 g (41,3 mMol) 5-Acetylamino-2,4,6-trijod-isophthalsäure-(2-hydroxy-1-hydroxymethyläthyl)-amidchlorid werden bei Raumtemperatur zu einer Lösung von 9,8 g (86,7 mMol) 2,3-Dihydroxy-N-methylpropylamin in 80 ml trockenem DMF gegeben und 3 Stunden bei Raumtemperatur gerührt. Die Reaktionslösung wird dann in 800 ml Methylenchlorid eingerührt, der Niederschlag abgetrennt, in Wasser gelöst und an Amberlite XAD-4 entsalzt. Ausbeute 18,5 g (24,3 mMol) = 59 % d.Th.

b) 5-(N-Acetyl-2-hydroxyäthylamino)-2,4,6-trijod-isophthalsäure-[(2,3-dihydroxy-N-methyl-propyl)-(2-hydroxy-1-hydroxymethyläthyl)]-diamid

10,6 g (13,9 mMol) 5-Acetylamino-2,4,6-trijod-isophthalsäure-[(2,3-dihydroxy-N-methyl-propyl)-(2-hydroxy-1-hydroxymethyläthyl)]-diamid werden in 8,4 ml (42 mMol) 5N NaOH gelöst, 3,5 g (28,2 mMol) Bromäthanol hinzugefügt und 5 Stunden bei Raumtemperatur gerührt. Die Reaktionslösung wird dann auf 50 ml verdünnt, mit Salzsäure auf pH 7 gestellt, eingedampft und der Rückstand in 50 ml Äthanol aufgenommen. Vom ungelösten Salzwird abgetrennt, die Äthanollösung eingeengt, der Rückstand in Wasser gelöst und an Amberlite XAD-4 entsalzt. Ausbeute : 7,5 g (9,3 mMol) = 67 % d.Th.

Beispiel 14

5-Acetylamino-2,4,6-trijod-isophthalsäure-[(tris-hydroxymethyl-methyl)-(2-hydroxy-1-hydroxymethyläthyl)]-diamid

25 g (32 mMol) 5-Acetylamino-2,4,6-trijod-isophthalsäure-(2-hydroxy-1-hydroxymethyläthyl)-amidchlorid und 38,8 g (320 mMol) Tris(hydroxymethyl)-methylamin werden in 500 ml Dioxan 8 Stunden am Rückfluss gekocht. Die Reaktionslösung wird dann eingedampft, der Rückstand in 100 ml DMF in der Wärme gelöst und die Lösung in 800 ml auf 90 °C erwärmtes Dioxan eingerührt. Dabei fällt das Hydrochlorid des Amins aus. Die Lösung wird dekantiert, eingedampft, in 300 ml Wasser aufgenommen, mit Salzsäure auf pH 7 eingestellt und an Amberlite XAD-4 entsalzt. Ausbeute : 17,5 g (22,5 mMol) = 70,4 % d.Th.

Beispiel 15

5-(N-Acetyl-2-hydroxyäthylamino)-2,4,6-trijod-isophthalsäure-[(2,3-dihydroxy-propyl)-(2,3-dihydroxy-N-methyl-propyl)]-diamid

a) 5-Acetylamino-2,4,6-trijod-isophthalsäure-[(2,3-dihydroxy-propyl)-(2,3-dihydroxy-N-methyl-propyl)]-diamid

44,4 g (50 mMol) 5-Amino-2,4,6-trijod-isophthalsäure-[(2,3-diacetoxy-propyl)-(2,3-diacetoxy-N-methyl-propyl)]-diamid werden in 100 ml DMA gelöst, bei 10 °C 7 g (89,3 mMol) Acetylchlorid eingetropft und 20 Stunden bei Raumtemperatur nachgerührt. Die Reaktionslösung wird dann eingedampft, das Öl in 100 ml Wasser suspendiert und mit 50 ml konz. wässrigem Ammoniak 5 Stunden bei Raumtemperatur gerührt, bis eine klare Lösung entstanden ist. Diese wird zu einem Öl eingedampft, das Öl in 500 ml Methylenchlorid eingerührt, der feste Niederschlag abfiltriert, getrocknet, in Wasser gelöst und an 1 lit. Adsorber Amberlite XAD-4 entsalzt. Ausbeute : 29 g (38 mMol) = 76 % d.Th.

b) 5-(N-Acetyl-2-hydroxyäthylamino)-2,4,6-trijod-isophthalsäure-[(2,3-dihydroxy-propyl)-(2,3-dihydroxy-N-methylpropyl)]-diamid

20 g (26,3 mMol) 5-Acetylamino-2,4,6-trijod-isophthalsäure-[(2,3-dihydroxy-propyl)-(2,3-dihydroxy-N-methyl-propyl)]-diamid werden in 40 ml (200 mMol) 5N NaOH gelöst, 6,6 g (53 mMol) Bromäthanol hinzugefügt und 5 Stunden bei Raumtemperatur gerührt. Die Reaktionslösung wird dann unter Kühlung mit Salzsäure neutralisiert, eingedampft, der Rückstand in Äthanol aufgenommen, vom unlöslichen Salz abfiltriert, das Filtrat eingedampft, in Wasser gelöst und an 800 ml Adsorber Amberlite XAD-4 entsalzt. Ausbeute : 12,7 g (15,75 mMol) = 63 % d.Th.

**Ansprüche** (für die Vertragsstaaten : BE, CH, FR, DE, GB, IT, LU, NL, SE)

1. Trijodierte Isophthalsäurediamide der allgemeinen Formel

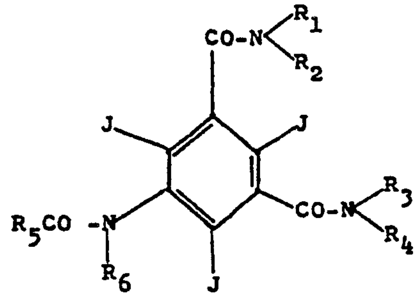

(I),

worin

die Amidreste —CO—N · $R_1R_2$ und —CO—N · $R_3R_4$ voneinander verschieden sind und

$R_1$ ein Wasserstoffatom oder einen niederen Alkylrest,

$R_2$ einen gerad- oder verzweigtkettigen Mono- oder Polyhydroxyalkylrest,

$R_3$ ein Wasserstoffatom oder einen niederen Alkylrest,

$R_4$ einen gerad- oder verzweigtkettigen Mono- oder Polyhydroxyalkylrest,

$R_5$ eine niedere Alkyl- oder eine niedere Hydroxyalkyl- oder eine niedere Alkoxy-niedere-alkylgruppe, und

$R_6$ ein Wasserstoffatom oder einen gegebenenfalls hydroxylierten niederen Alkylrest

bedeuten.

2. 5-Methoxyacetylamino-2,4,6-trijod-isophthalsäure-[(2,3-dihydroxy-N-methyl-propyl)-(2,3-dihydroxypropyl)]-diamid.

3. 5-(S-2-Hydroxypropionylamino)-2,4,6-trijod-isophthalsäure-[(2,3-dihydroxy-N-methyl-propyl)-(2,3-dihydroxypropyl)]-diamid.

4. 5-(N-Acetyl-2-hydroxyäthylamino)-2,4,6-trijod-isophthalsäure-[(2,3-dihydroxypropyl)-(2,3-dihydroxy-N-methyl-propyl)]-diamid.

5. 5-Methoxyacetylamino-2,4,6-trijod-isophthalsäure-[(2,3-dihydroxy-N-methyl-propyl)-(2-hydroxy-1-hydroxymethyl-äthyl)]-diamid.

6. 5-Acetylamino-2,4,6-trijod-isophthalsäure-[(2,3-dihydroxy-propyl)-(2,3-dihydroxy-N-methyl-propyl)]-diamid.

7. Verfahren zur Herstellung von Verbindungen der allgemeinen Formel I, dadurch gekennzeichnet, dass man in an sich bekannter Weise

a) eine Verbindung der allgemeinen Formel IIA

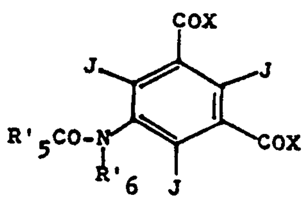

(IIA),

worin

$R'_5 = R_5$, wobei jedoch im Alkylrest anwesende freie Hydroxylgruppen gegebenenfalls funktionell abgewandelt sein können,

$R'_6 = R_6$, wobei jedoch im Alkylrest anwesende freie. Hydroxylgruppen gegenenfalls funktionell abgewandelt sein können,

X = ein reaktiver Säure- oder Ester-Rest bedeuten, zunächst, mit einer Base der Formel III

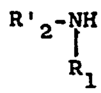

(III),

worin

$R_1$ Wasserstoff oder einen niederen Alkylrest,

$R'_2 = R_2$, wobei im Alkylrest anwesende freie Hydroxylgruppen gegebenenfalls funktionell abgewandelt sein können,

bedeuten, und das so erhaltene 5-[$R'_5$—CO($R'_6$)-amino]-2,4,6-trijod-isophthalsäure-($R'_2$—N—$R_1$)-amidchlorid dann mit einer Base der Formel IV

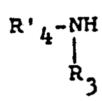

(IV),

worin

$R_3$ Wasserstoff oder einen niederen Alkylrest,

$R'_4 = R_4$, wobei im Alkylrest anwesende freie Hydroxylgruppen gegebenenfalls funktionell abgewandelt sein können,

bedeuten, umsetzt und gegebenenfalls anschliessend die aromatische Acylaminogruppe mit einem $R'_6$-enthaltenden Alkylierungsmittel ($R'_6 = R_6$, worin anwesende freie Hydroxylgruppen funktionell geschützt sind) N-alkyliert und/oder geschützte Hydroxylgruppen verseift, oder

b) eine Verbindung der allgemeinen Formel IIB

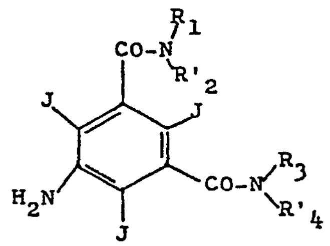

(IIB),

worin

$R_1$ und $R_3$ das gleiche wie oben und $R'_2$ und $R'_4 = R_2$ und

$R_4$, wobei jedoch im Alkylrest anwesende freie Hydroxylgruppen funktionell abgewandelt sein können, bedeuten, mit einem reaktiven $R'_5$-Carbonsäurederivat ($R'_5 = R_5$ worin anwesende freie Hydroxylgruppen funktionell geschützt sind) N-acyliert, und gegebenenfalls anschliessend mit einem $R'_6$-enthaltenden Alkylierungsmittel N-alkyliert und/oder geschützte Hydroxylgruppen in Freiheit setzt, oder

c) eine Verbindung der allgemeinen Formel IIC

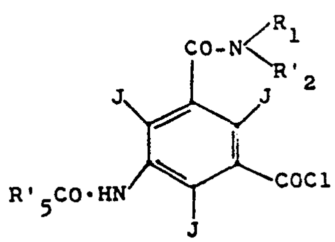

(IIC),

worin

$R_1$, $R'_2$ und $R'_5$ die oben genannte Bedeutung haben, mit einer Base der Formel IV umsetzt und gegebenenfalls anschliessend mit einem $R'_6$-enthaltenden Alkylierungsmittel N-alkyliert und/oder geschützte Hydroxylgruppen in Freiheit setzt.

8. Röntgenkontrastmittel enthaltend Verbindungen gemäss Anspruch 1-6 als schattengebende Substanz.

**Ansprüche** (für Österreich)

1. Verfahren zur Herstellung von Verbindungen der allgemeinen Formel I,

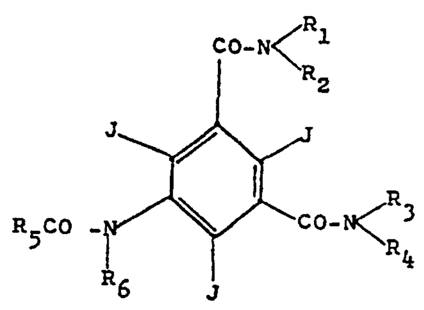

(I),

worin

die Amidreste —CO—N · $R_1R_2$ und —CO—N · $R_3R_4$ voneinander verschieden sind und

$R_1$ ein Wasserstoffatom oder einen niederen Alkylrest,

$R_2$ einen gerad- oder verzweigtkettigen Mono- oder Polyhydroxyalkylrest,

$R_3$ ein wasserstoffatom oder einen niederen Alkylrest,

$R_4$ einen gerad- oder verzweigtkettigen Mono- oder Polyhydroxyalkylrest,

$R_5$ eine niedere Alkyl- oder eine niedere Hydroxyalkyl- oder eine niedere Alkoxy-niedere-alkylgruppe, und

$R_6$ ein Wasserstoffatom oder einen gegebenenfalls hydroxylierten niederen Alkylrest beudeuten, dadurch gekennzeichnet, daß man in an sich bekannter Weise

a) eine Verbindung der allgemeinen Formel IIA

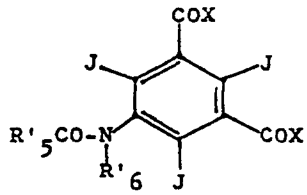

(IIA),

worin

$R'_5 = R_5$, wobei jedoch im Alkylrest anwesende freie Hydroxylgruppen gegebenenfalls funktionell abgewandelt sein können,

$R'_6 = R_6$, wobei jedoch im Alkylrest anwesende freie Hydroxylgruppen gegebenenfalls funktionell abgewandelt sein können,

X = ein reaktiver Säure- oder Ester-Rest bedeuten, zunächst mit einer Base der Formel III

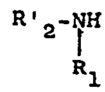

(III),

worin

$R_1$ Wasserstoff oder einen niederen Alkylrest,

$R'_2 = R_2$, wobei im Alkylrest anwesende freie Hydroxylgruppen gegebenenfalls funktionell abgewandelt sein können,

bedeuten, und das so erhaltene 5-[$R'_5$—CO($R'_6$)-amino]-2,4,6-trijod-isophthalsäure-($R'_2$—N—$R_1$)-amidchlorid dann mit einer Base der Formel IV

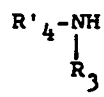

(IV),

worin

$R_3$ Wasserstoff oder einen niederen Alkylrest,

$R'_4 = R_4$, wobei im Alkylrest anwesende freie Hydroxylgruppen gegebenenfalls funktionell abgewandelt sein können,

bedeuten, umsetzt und gegebenenfalls anschließend die aromatische Acylaminogruppe mit einem $R'_6$-enthaltenden Alkylierungsmittel ($R'_6 = R_6$, worin anwesende freie Hydroxylgruppen funktionell geschützt sind) N-alkyliert und/oder geschützte Hydroxylgruppen verseift, oder

b) eine Verbindung der allgemeinen Formel IIB

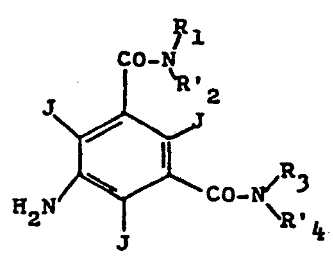

(IIB),

worin

$R_1$ und $R_3$ das gleiche wie oben und $R'_2$ und $R'_4 = R_2$ und

$R_4$, wobei jedoch im Alkylrest anwesende freie Hydroxylgruppen funktionell abgewandelt sein können, bedeuten, mit einem reaktiven $R'_5$-Carbonsäurederivat ($R'_5 = 5$, worin anwesende freie Hydroxylgruppen funktionell geschützt sind) N-acyliert, und gegebenenfalls anschliessend mit einem $R'_6$- enthaltenden Alkylierungsmittel N-alkyliert und/oder geschützte Hydroxylgruppen in Freiheit setzt, oder

c) eine Verbindung der allgemeinen Formel IIC

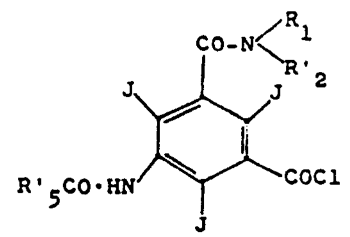

(IIC),

worin

$R_1$, $R'_2$ und $R'_5$ die oben genannte Bedeutung haben, mit einer Base der Formel IV umsetzt und gegebenenfalls anschliessend mit einem $R'_6$-enthaltenden Alkylierungsmittel N-alkyliert und/oder geschützte Hydroxylgruppen in Freiheit setzt.

2. Röntgenkontrastmittel, gekennzeichnet, durch einen Gehalt an trijodierten Isophthalsäurediamiden der allgemeinen Formel I als schattengebende Substanz.

3. Röntgenkontrastmittel nach Anspruch 2, gekennzeichnet durch einen Gehalt an 5-Methoxyacetylamino-2,4,6-trijod-isophthalsäure-[(2,3-dihydroxy-N-methyl-propyl)-(2,3-dihydroxypropyl)]-diamid als schattengebende Substanz.

4. Röntgenkontrastmittel nach Anspruch 2, gekennzeichnet durch einen Gehalt an 5-(S-2-Hydroxypropionylamino)-2,4,6-trijod-isophthalsäure-[(2,3-dihydroxy-N-methyl-propyl)-(2,3-dihydroxypropyl)]-diamid als schattengebende Substanz.

5. Röntgenkontrastmittel nach Anspruch 2, gekennzeichnet durch einen Gehalt an 5-(N-Acetyl-2-hydroxyäthylamino)-2,4,6-trijod-isophthalsäure-[(2,3-dihydroxypropyl)-(2,3-dihydroxy-N-methylpropyl)]-diamid als schattengebende Substanz.

6. Röntgenkontrastmittel nach Anspruch 2, gekennzeichnet durch einen Gehalt an 5-Methoxyacetylamino-2,4,6-trijod-isophthalsäure-[(2,3-dihydroxy-N-methyl-propyl)-(2-hydroxy-1-hydroxymethyläthyl)]-diamid als schattengebende Substanz.

7. Röntgenkontrastmittel nach Anspruch 2, gekennzeichnet durch einen Gehalt an 5-Acetylamino-2,4,6-trijod-isophthalsäure-[(2,3-dihydroxy-propyl)-(2,3-dihydroxy-N-methyl-propyl)]-diamid als schattengebende Substanz.

**Claims** (for the contracting States : BE, CH, FR, DE, GB, IT, LU, NL, SE)

1. Triiodised isophthalic acid diamides of the general formula

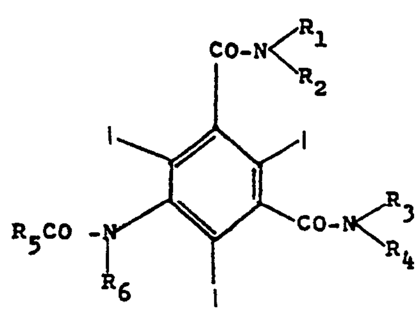

(I),

in which

the amide radicals $—CO—N \cdot R_1R_2$ and $—CO—N \cdot R_3R_4$ are different from each other, and

$R_1$ represents a hydrogen atom or a lower alkyl radical,

$R_2$ represents a straight-chain or branched mono- or polyhydroxyalkyl radical,

$R_3$ represents a hydrogen atom or a lower alkyl radical,

$R_4$ represents a straight-chain or branched mono- or polyhydroxyalkyl radical,

$R_5$ represents a lower alkyl group, a lower hydroxyalkyl group or a lower alkoxy-lower alkyl group, and

$R_6$ represents a hydrogen atom or an optionally hydroxylated lower alkyl radical.

2. 5-methoxyacetylamino-2,4,6-triiodoisophtalic acid [(2,3-dihydroxy-N-methylpropyl)-(2,3-dihydroxypropyl)]-diamide.

3. 5-(S-2-hydroxypropionylamino)-2,4,6-triiodoisophtalic acid [(2,3-dihydroxy-N-methylpropyl)-(2,3-dihydroxypropyl)]-diamide.

4. 5-(N-acetyl-2-hydroxyethylamino)-2,4,6-triiodoisophthalic acid [(2,3-dihydroxypropyl)-(2,3-dihydroxy-N-methylpropyl)]-diamide.

5. 5-methoxyacetylamino-2,4,6-triiodoisophthalic acid [(2,3-dihydroxy-N-methylpropyl)-(2-hydroxy-1-hydroxymethylethyl)]-diamide.

6. 5-acetylamino-2,4,6-triiodoisophthalic acid [(2,3-dihydroxypropyl)-(2,3-dihydroxy-N-methylpropyl)]-diamide.

7. Process for the manufacture of compounds of the general formula I, characterised in that, in a manner known *per se*,

a) a compound of the general formula IIA

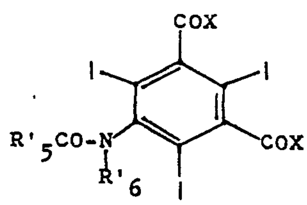

(IIA)

in which

$R'_5 = R_5$, wherein, however, free hydroxy groups present in the alkyl radical may be optionally functionally modified,

$R'_6 = R_6$, wherein, however, free hydroxy groups present in the alkyl radical may be optionally functionally modified, and

X represents a reactive acid radical or a reactive ester radical,

is reacted first with a base of the formula III

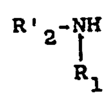

(III),

in which

$R_1$ represents hydrogen or a lower alkyl radical, and

$R'_2 = R_2$, wherein the free hydroxy groups present in the alkyl radical may be optionally functionally modified,

and the 5-[$R'_5$—CO($R'_6$)—amino]-2,4,6-triiodoisophthalic acid ($R'_2$—N—$R_1$)-amide chloride thus obtained is then reacted with a base of the formula IV

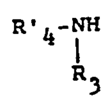

(IV),

in which

$R_3$ represents hydrogen or a lower alkyl radical, and

$R'_4 = R_4$, wherein free hydroxy groups present in the alkyl radical may be optionally functionally modified,

and optionally subsequently the aromatic acylamino group is N-alkylated with an alkylating agent containing $R'_6$ ($R'_6 = R_6$, wherein free hydroxy groups present are functionally protected), and/or protected hydroxy groups are hydrolysed, or

b) a compound of the general formula IIB

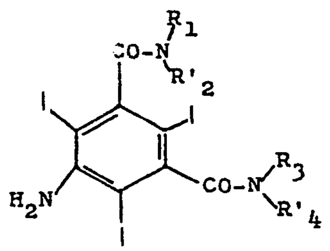

(IIB),

in which

$R_1$ and $R_3$ have the same meaning as given above, and

$R'_2$ and $R'_4 = R_2$ and $R_4$, wherein, however, free hydroxy groups present in the alkyl radical may be functionally modified, is N-acylated with a reactive $R'_5$-carboxylic acid derivative ($R'_5 = R_5$ in which free hydroxy groups present are functionally protected), and optionally subsequently N-alkylated with an alkylating agent containing $R'_6$, and/or protected hydroxy groups are liberated, or

c) a compound of the general formula IIC

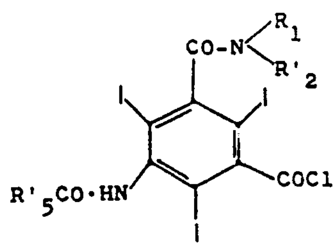

(IIC),

in which

$R_1$, $R'_2$ and $R'_5$ have the meanings given above, is reacted with a base of the formula IV and optionally subsequently N-alkylated with an alkylating agent containing $R'_6$, and/or protected hydroxy groups are liberated.

8. X-ray contrast media containing compounds according to claims 1 to 6 as radio-opaque substance.

**Claims** (for Austria)

1. Process for the manufacture of compounds of the general formula I,

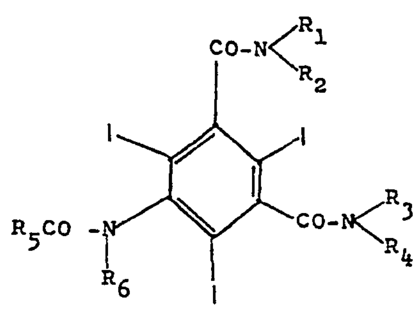

(I),

in which

the amide radicals —CO—N · $R_1R_2$ and —CO—N · $R_3R_4$ are different from each other, and

$R_1$ represents a hydrogen atom or a lower alkyl radical,

$R_2$ represents a straight-chain or branched mono- or polyhydroxyalkyl radical,

$R_3$ represents a hydrogen atom or a lower alkyl radical,

$R_4$ represents a straight-chain or branched mono- or polyhydroxyalkyl radical,

$R_5$ represents a lower alkyl group, a lower hydroxyalkyl group or a lower alkoxy-lower alkyl group, and

$R_6$ represents a hydrogen atom or an optionally hydroxylated lower alkyl radical, characterised in that, in a manner known *per se*,

a) a compound of the general formula IIA

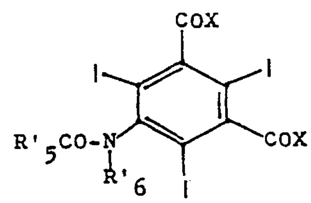

(IIA),

in which

$R'_5 = R_5$, wherein, however, free hydoxy groups present in the alkyl radical may be optionally functionally modified,

$R'_6 = R_6$, wherein, however, free hydroxy groups present in the alkyl radical may be optionally functionally modified, and

X represents a reactive acid radical or a reactive ester radical, is reacted first with a base of the formula III

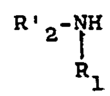

(III),

in which

$R_1$ represents hydrogen or a lower alkyl radical, and

$R'_2 = R_2$, wherein the free hydroxy groups present in the alkyl radical may be optionally functionally modified,

and the 5—[$R'_5$—CO($R'_6$)-amino]—2,4,6-triiodoisophthalic acid ($R'_2$—N—$R_1$)-amide chloride thus obtained is then reacted with a base of the formula IV

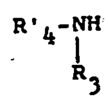

(IV),

in which

$R_3$ represents hydrogen or a lower alkyl radical, and $R'_4 = R_4$, wherein free hydroxy groups present in the alkyl radical may be optionally functionally modified,

and optionally subsequently the aromatic acylamino group is N-alkylated with an alkylating agent containing $R'_6$($R'_6 = R_6$, wherein free hydroxy groups present are functionally protected), and/or protected hydroxy groups are hydrolysed, or

b) a compound of the general formula IIB

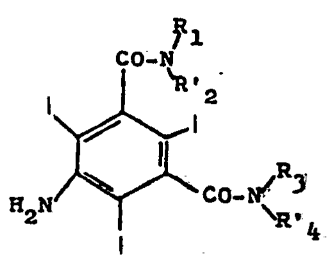

(IIB),

in which

$R_1$ and $R_3$ have the same meaning as given above, and $R'_2$ and $R'_4 = R_2$ and $R_4$, wherein, however, free hydroxy groups present in the alkyl radical may be functionally modified, is N-acylated with a reactive $R'_5$-carboxylic acid derivative ($R'_5 = R_5$ in which free hydroxy groups present are functionally protected), and optionally subsequently N-alkylated with an alkylating agent containing $R'_6$, and/or

protected hydroxy groups are liberated, or

c) a compound of the general formula IIC

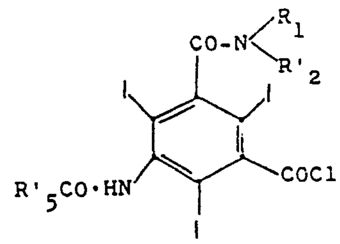

(IIC),

in which

$R_1$, $R'_2$ and $R'_5$ have the meanings given above, is reacted with a base of the formula IV and optionally subsequently N-alkylated with an alkylating agent containing $R'_6$, and/or protected hydroxy groups are liberated.

2. X-ray contrast agents containing compounds of the general formula I as radio-opaque substance.

3. X-ray contrast agent according to claim 2 containing 5-methoxyacetylamino-2,4,6-triiodoisophthalic acid [(2,3-dihydroxy-N-methylpropyl)-(2,3-dihydroxypropyl)]-diamide as radio-opaque substance.

4. X-ray contrast agent according to claim 2 containing 5-(S-2-hydroxypropionylamino)-2,4,6-triiodoisophthalic acid[(2,3-dihydroxy-N-methylpropyl)-(2,3-dihydroxypropyl)]-diamide as radio-opaque substance.

5. X-ray contrast agent according to claim 2 containing 5-(N-acetyl-2-hydroxyethylamino)-2,4,6-triiodoisophthalic acid [(2,3-dihydroxypropyl)-(2,3-dihydroxy-N-methylpropyl)]-diamide as radio-opaque substance.

6. X-ray contrast agent according to claim 2 containing 5-methoxyacetylamino-2,4,6-triiodoisophthalic acid [(2,3-dihydroxy-N-methylpropyl)-(2-hydroxy-1-hydroxymethylethyl)]-diamide as radio-opaque substance.

7. X-ray contrast agent according to claim 2 containing 5-acetylamino-2,4,6-triiodoisophthalic acid [(2,3-dihydroxy-propyl)-(2,3-dihydroxy-N-methylpropyl)]-diamide as radio-opaque substance.

**Revendications** (pour les Etats contractants : BE, CH, FR, DE, GB, IT, LU, NL, SE)

1. Les diamides de l'acide isophtalique tri-iodés de formule générale

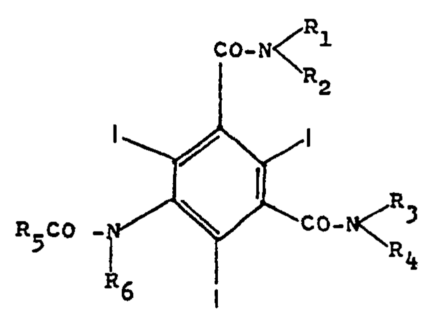

(I),

dans laquelle

les radicaux amides —CO—N—$R_1R_2$ et —CO—N—$R_3$—$R_4$ sont différents l'un de l'autre et

$R_1$ désigne un atome d'hydrogène ou un alkyle inférieur,

$R_2$ un radical mono- ou poly-hydroxy-alkylique à chaîne droite ou ramifiée,

$R_3$ un atome d'hydrogène ou un alkyle inférieur,

$R_4$ un radical mono- ou poly-hydroxy-alkylique à chaîne droite ou ramifiée,

$R_5$ un alkyle inférieur, un hydroxy-alkyle inférieur ou un alcoxy-alkyle à groupes alcoxy et alkyle inférieurs, et

$R_6$ désigne un atome d'hydrogène ou un alkyle inférieur éventuellement hydroxylé.

2. [(2,3-Dihydroxy-N-méthyl-propyl)-(2,3-dihydroxypropyl)]-diamide de l'acide 5-méthoxyacétylamino-2,4,6-tri-iodo-isophtalique.

3. [(2,3-Dihydroxy-N-méthyl-propyl)-(2,3-dihydroxypropyl)]-diamide de l'acide 5-(S-2-hydroxypropionylamino)-2,4,6-tri-iodo-isophtalique.

4. [(2,3-Dihydroxypropyl)-(2,3-dihydroxy-N-méthylpropyl)]-diamide de l'acide 5-(N-acétyl-2-hydroxyéthylamino)-2,4,6-tri-iodo-isophtalique.

5. [(2,3-Dihydroxy-N-méthyl-propyl)-(2-hydroxy-1-hydroxyméthyléthyl)]-diamide de l'acide 5-méthoxyacétylamino-2,4,6-tri-iodo-isophtalique.

6. [(2,3-Dihydroxy-propyl)-(2,3-dihydroxy-N-méthyl-propyl)]-diamide de l'acide 5-acétylamino-2,4,6-tri-iodo-isophtalique.

7. Procédé de préparation des composés de formule générale I selon la revendication 1, caractérisé en ce que, ou bien

a) on fait réagir un composé de formule générale IIA

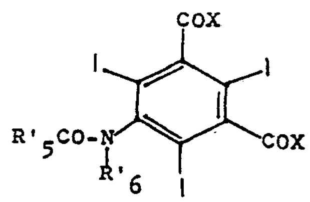

(IIA),

dans laquelle

$R'_5 = R_5$, mais cependant des hydroxyles libres éventuellement présents sur l'alkyle peuvent être le cas échéant fonctionnellement modifiés,

$R'_6 = R_6$, mais des hydroxyles libres éventuellement présents peuvent être aussi le cas échéant fonctionnellement modifiés, et

X désigne un radical d'acide ou d'ester réactif,

avec une base de formule III

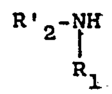

(III)

dans laquelle

$R_1$ représente l'hydrogène ou un alkyle inférieur, et

$R'_2 = R_2$, mais des hydroxyles libres sur l'alkyle peuvent être éventuellement fonctionnellement modifiés,

puis on fait réagir le chlorure de l'amide ($R'_2$—N—$R_1$) 5-[$R'_5$-CO($R'_6$)-amino]-2,4,6-triiodo-isophtalique ainsi formé avec une base de formule IV

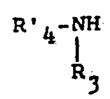

(IV)

dans laquelle

$R_3$ représente l'hydrogène ou un alkyle inférieur, et

$R'_4 = R_4$, mais des hydroxyles libres sur l'alkyle pouvant être éventuellement fonctionnellement modifiés,

puis le cas échéant, on alkyle sur l'azote le groupe acylamino aromatique avec un agent alkylant apportant le radical $R'_6$($R'_6 = R_6$, où des hydroxyles libres présents sont fonctionnellement protégés) et/ou on saponifie des hydroxyles protégés, ou bien

b) on acyle sur l'azote, avec un dérivé réactif d'un acide $R'_5$-carboxylique ($R'_5$=$R_5$, où des hydroxyles libres présents sont fonctionnellement protégés), un composé de formule générale IIB

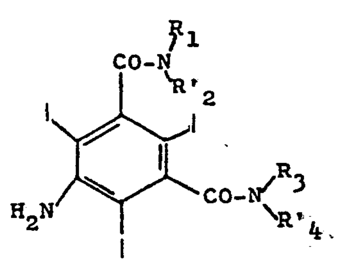

(IIB),

dans laquelle

$R_1$ et $R_3$ ont les mêmes significations que ci-dessus et $R'_2$ et $R'_4=R_2$ et $R_4$, mais toutefois des hydroxyles libres sur l'alkyle pouvant être fonctionnellement modifiés, puis éventuellement on l'alkyle sur l'azote avec un agent alkylant apportant le radical $R'_6$ et/ou on libère des hydroxyles protégés, ou encore

c) on fait réagir un composé de formule générale IIC

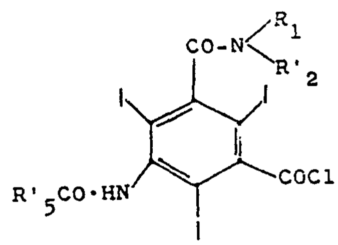

(IIC),

dans laquelle

$R_1$, $R'_2$ et $R'_5$ ont les mêmes significations que ci-dessus, avec une base de formule IV, puis éventuellement on alkyle l'atome d'azote avec un agent alkylant apportant le radical $R'_6$ et/ou on libère des hydroxyles protégés.

8. Agents de contraste pour rayons X, comprenant des composés selon l'une quelconque des revendications 1 à 6 comme substances opacifiantes.

**Revendications** (pour l'Autriche)

1. Procédé de préparation des composés de formule générale I,

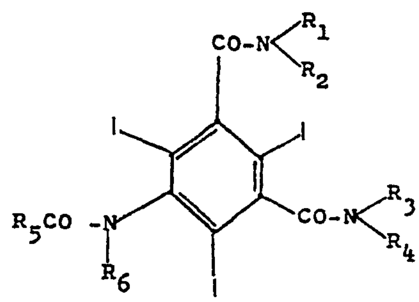

(I),

dans laquelle

les radicaux amides —CO—N—$R_1R_2$ et —CO—N—$R_3R_4$ sont différents l'un de l'autre et

$R_1$ désigne un atome d'hydrogène ou un alkyle inférieur,

$R_2$ un radical mono- ou poly-hydroxy-alkylique à chaîne droite ou ramifiée,

$R_3$ un atome d'hydrogène ou un alkyle inférieur,

$R_4$ un radical mono- ou poly-hydroxy-alkylique à chaîne droite ou ramifiée,

$R_5$ un alkyle inférieur, un hydroxy-alkyle inférieur ou un alkoxy-alkyle à groupes alcoxy et alkyle inférieurs, et

$R_5$ désigne un atome d'hydrogène ou un alkyle inférieur éventuellement hydroxylé,

caractérisé en ce que, ou bien

a) on fait réagir un composé de formule générale IIA

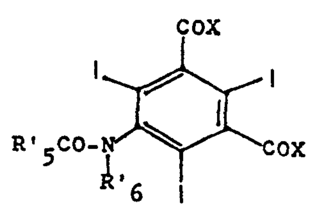

(IIA),

dans laquelle

$R'_5 = R_5$, mais cependant des hydroxyles libres éventuellement présents sur l'alkyle peuvent être le cas échéant fonctionnellement modifiés,

$R'_6=R_6$, mais des hydroxyles libres éventuellement présents peuvent être aussi le cas échéant fonctionnellement modifiés, et

X désigne un radical d'acide ou d'ester réactif,

avec une base de formule III

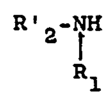

(III),

dans laquelle

$R_1$ représente l'hydrogène ou un alkyle inférieur, et

$R'_2 = R_2$, mais des hydroxyles libres sur l'alkyle peuvent être éventuellement fonctionnellement modifiés,

puis on fait réagir le chlorure de l'amide ($R'_2$—N—$R_1$) 5-[$R'_5$—CO($R'_6$)-amino]-2,4,6-triiodo-isophtalique ainsi formé avec une base de formule IV

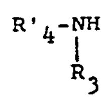

(IV),

dans laquelle

$R_3$ représente l'hydrogène ou un alkyle inférieur, et

$R'_4=R_4$, mais des hydroxyles libres sur l'alkyle pouvant être éventuellement fonctionnellement modifiés,

puis et cas échéant, on alkyle sur l'azote le groupe acylamino aromatique avec un agent alkylant apportant le radical $R'_6$($R'_6=R_6$, où des hydroxyles libres présents sont fonctionnellement protégés) et/ou on saponifie des hydroxyles protégés, ou bien

b) on acyle sur l'azote, avec un dérivé réactif d'un acide $R'_5$-carboxylique ($R'_5=R_5$, où des hydroxyles libres présents sont fonctionnellement protégés), un composé de formule générale IIB

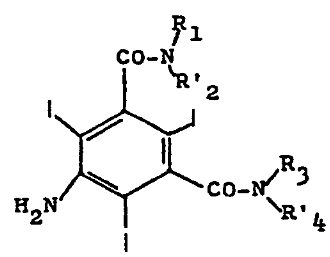

(IIB),

dans laquelle

$R_1$ et $R_3$ ont les mêmes significations que ci-dessus et $R'_2$ et $R'_4=R_2$ et $R_4$, mais toutefois des hydroxyles libres sur l'alkyle pouvant être fonctionnellement modifiés, puis éventuellement on l'alkyle sur l'azote avec un agent alkylant apportant le radical $R'_6$ et/ou on libère des hydroxyles protégés, ou encore

c) on fait réagir un composé de formule générale IIC

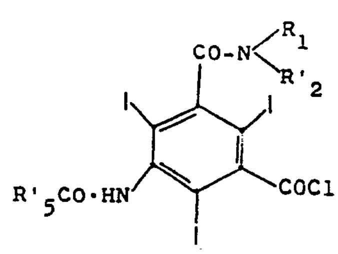

(IIC),

dans laquelle

$R_1$, $R'_2$ et $R'_5$ ont les mêmes significations que ci-dessus, avec une base de formule IV, puis

éventuellement on alkyle l'atome d'azote avec un agent alkylant apportant le radical $R'_6$ et/ou on libère des hydroxyles protégés.

2. Agents de contraste radiologiques, caractérisés en ce qu'ils contiennent, comme substances opacifiantes, des diamides de l'acide isophtalique tri-iodés de formule générale I.

3. Agents de contraste radiologiques selon la revendication 2, caractérisés en ce qu'ils contiennent, comme substances opacifiantes, des [(2,3-Dihydroxy-N-méthyl-propyl)-(2,3-dihydroxypropyl)]-diamide de l'acide 5-méthoxyacétylamino-2,4,6-tri-iodo-isophtalique.

4. Agents de contraste radiologiques selon la revendication 2, caractérisés en ce qu'ils contiennent, comme substances opacifiantes, des [(2,3-Dihydroxy-N-méthyl-propyl)-(2,3-dihydroxy-propyl)]-diamide de l'acide 5-(S-2-hydroxypropionylamino)-2,4,6-tri-iodo-isophtalique.

5. Agents de contraste radiologiques selon la revendication 2, caractérisés en ce qu'ils contiennent, comme substances opacifiantes, des [(2,3-Dihydroxypropyl)-(2,3-dihydroxy-N-méthylpropyl)]-diamide de l'acide 5-N-acétyl-2-hydroxyéthylamino)-2,4,6-tri-iodo-isophtalique.

6. Agents de contraste radiologiques selon la revendication 2, caractérisés en ce qu'ils contiennent, comme substances opacifiantes, des [(2,3-Dihydroxy-N-méthyl-propyl)-(2-hydroxy-1-hydroxyméthyléthyl)]-diamide de l'acide 5-méthoxy-acétylamino-2,4,6-tri-iodo-isophtalique.

7. Agents de contraste radiologiques selon la revendication 2, caractérisés en ce qu'ils contiennent, comme substances opacifiantes, des [(2,3-Dihydroxy-propyl)-(2,3-dihydroxy-N-méthyl-propyl)]-diamide de l'acide 5-acétylamino-2,4,6-tri-iodo-isophtalique.